# EUROPEAN PATENT SPECIFICATION

(11) **EP 0 943 919 B1**
(45) Date of publication and mention of the grant of the patent: **15.10.2003**
(21) Application number: 99302129.4
(22) Date of filing: 19.03.1999
(51) Int. Cl.: G01N 33/543, G01N 33/82

(54) **An assay surface that permits an analyte releasing step**
Eine Testoberfläche die einen Analytentbindungschritt erlaubt
Une surface d'essai qui permet une étape de libération d'analyte

(30) Priority: 20.03.1998 GB 9806055
(43) Date of publication of application: 22.09.1999
(73) Proprietor: Ortho-Clinical Diagnostics, Amersham, Buckinghamshire HP7 OHJ (GB)
(72) Inventor: Edwards, Steve, Aylesbury, Bucks HP8 1HY (GB); Sefton, Joanna, London SW17 8DQ (GB); Hipkiss, Jayne Beverley, Newbury, Berkshire RG13 2BL (GB); Edgar, Heather Anne, Hertfordshire HP23 4AY, London (GB); Dawkes, Adrian Charles, Windsor, Berkshire SL4 3JU (GB)
(74) Representative: Mercer, Christopher Paul

(56) References cited:
- EP-A- 0 348 174
- WO-A-94/03530
- DE-A- 19 530 078
- US-A- 5 629 213
- B. L. FREY ET AL.: "Control of the specific adsorption of proteins onto gold surfaces with Poly(L-lysine) monolayers" ANALYTICAL CHEMISTRY., vol. 67, no. 24, 15 December 1995, pages 4452-4457, XP002107496 COLUMBUS US
- CHEMICAL ABSTRACTS, vol. 128, no. 1, 5 January 1998 Columbus, Ohio, US; abstract no. 1649, K. CHOI ET AL.: "Efficient biotinylation of nitrocellulose membrane for immuno-filtration capture assay " page 185; column 1; XP002107498 & J. BIOCHEM. MOL. BIOL., vol. 30, no. 5, 1997, pages 308-314,
- R. H. CHRISTENSON EZT AL.: "Two radioassays for serum vitamin B12 and folate determination compared in a reference interval study" CLINICAL CHEMISTRY., vol. 31, no. 8, August 1985, pages 1358-1360, XP002107497 WINSTON US

## Description

### Background of the Invention

The present invention relates to the use of a surface or solid phase in an assay.

WO 94/03530 describes a method of coupling on solid phases in which an activated dextran is coupled with a resin.

The detection and quantification of various analytes in a sample of body fluid using immunoassay and other binding protein assay techniques has been well established in fields involving the diagnosis, treatment and monitoring of disease. Analytes can be any of various factors such as proteins, hormones, chemicals and their metabolic products. A commonly used binding protein assay technique involves the immobilization of a receptor for the analyte on a reaction vessel, for example a microwell. The immobilization can be accomplished by physical adsorption, chemical coupling or by utilizing specific binding pairs such as biotin and avidin [US Patent No. 5,367,624]. Some samples require pretreatment in order to release the analyte in a form that will allow the interaction of the analyte with the analyte-specific active surface and thus enable detection of the analyte. Routinely, assays where harsh sample pretreatment for releasing an analyte is required are performed in two separate vessels or on two physically separate surfaces. Hereinafter, such a pretreatment step is referred to as an analyte releasing step.

For example, in order to measure vitamin B12 or folate in a serum or plasma sample, it is necessary to release any proportion of the analyte that might be complexed to a binding or carrier protein or other agent such that the analyte specific assay components can bind the analyte in question and appropriate measurements can be made. Release of the bound or complexed analyte is routinely achieved by performing an analyte releasing step, for example, by adding a strong alkali to the sample or boiling the sample in the presence of a reducing agent, carried out in advance of the assay. [Branagan (1991)].

A problem that arises, however, is that the reagent used in the analyte releasing step may react with an active component on the microwell surface resulting in a decrease or elimination of analyte binding. Therefore, sample pretreatments or analyte releasing steps are routinely carried out in a separate vessel using an uncoated or non-activated vessel and a proportion of sample transferred to the active reaction vessel after the analyte releasing step, thus requiring an extra step in the assay procedure. Alternatively, the assay may be performed on a separate surface which is introduced into the sample holding vessel after releasing of the analyte.

### Summary of the Invention

One object of the present invention is to provide the use of an activated surface that is resistant to an analyte releasing step. The present invention therefore eliminates the need for separate vessels or surfaces and permits carrying out an analyte releasing step, which would otherwise denature or degrade the active components on the assay surface. Therefore, the present invention relates to the use of an assay surface, comprising: a support coated with a molecule coupled to biotin or a biotin mimic to form a complex, wherein the complex is resistant to an analyte releasing step. Preferably the molecule that is coupled to biotin or biotin mimic is a polyamine. Most preferably the polyamine is polylysine.

In another embodiment of the invention, the biotin or biotin mimic is further coupled to a biotin binding molecule selected from the group consisting of avidin and streptavidin. Either avidin or streptavidin can be used and one skilled in the art would know which one to employ depending on the assay conditions. An example of this embodiment would be a solid phase coated with polylysine coupled to biotin coupled to avidin.

Applicants' invention thus relates to a method for determining the presence of an analyte in a sample wherein an analyte releasing step is employed, comprising providing a surface comprising a support coated with a molecule coupled to biotin or a biotin mimic to form a complex, wherein the complex is resistant to the analyte releasing step.

### Detailed Description of The Invention

An assay surface is coated with a molecule, for example, a protein or polysaccharide, which is chemically coupled to biotin or a biotin mimic by standard coupling chemistry. The resulting complex can be further coupled to a biotin binding molecule such as avidin or streptavidin. For example, the avidin or streptavidin could be overcoated onto the solid phase or could be present in solution in an assay reagent. An assay could then be carried out where one or more analyte-specific components of the assay has been biotinylated.

The invention relates to a method for an assay using such a surface. As stated above, a commonly used binding protein assay technique involves the immobilization of a receptor for an analyte on a solid support. A typical technique might then comprise contacting the solid phase with a sample that may contain the analyte, contacting the solid phase with a second binding ligand for the analyte wherein said second ligand is labeled directly or indirectly with a detectable group and measuring the amount of the detectable group bound to the solid phase. Alternatively, the amount of detectable group not bound to the solid phase can be measured as an indication of the presence of the analyte. The detectable group can be, for example, an enzyme, a radioactive atom, a fluorescent molecule or a luminescent molecule. It will be understood by one of ordinary skill in the art that above-steps can be done sequentially or simultaneously.

The assay surface can be any support, for example, a microwell, gel, membrane, particles, beads or a dipstick. One skilled in the art would know what type of support best suits the assay to be performed. Coating techniques are known in the art and include, for example, physical adsorption or chemical binding. The term polyamine means a polymeric compound containing pendant amine groups, for example polylysine. A biotin mimic is any compound capable of binding to avidin or streptavidin. The high affinity interaction of biotin and avidin or streptavidin and its use in immunoassay and other immunologic techniques are well known in the art [Bayer, E.A. and Wilchek, M. (1988)].

The effectiveness and advantages of the invention are further illustrated by the following examples.

### Example 1

### Coating of microwells with polylysine - biotin/avidin

In one embodiment of the invention, the polyamine used to coat the microwells is polylysine. A polylysine-biotin conjugate, prepared by reaction of poly-L-lysine hydrochloride with biotin-XX-NHS (Calbiochem, Beeston, U.K.), was added to a polystyrene microwell at 2.5mg/mL in 0.1M sodium phosphate buffer pH 7.0 or 0.1M carbonate/bicarbonate buffer pH 10.0. The microwell was incubated at room temperature for sufficient time for the polylysine-biotin to saturate the surface. In this experiment, 6 minutes or greater was found to be sufficient. After washing the microwell, avidin was added at a concentration of 10mg/mL in the same phosphate or carbonate buffer. The microwell was incubated with the avidin solution for sufficient time to saturate the available biotin on the microwell. In this case, 50 minutes or greater was found to be sufficient. Concentrations of polyamine-biotin and avidin are used such that the resultant biotin binding capacity of the surface is 0.05 - 0.2 ng/mm². Biotin binding capacities can be measured by methods known in the art (US Patent 5,362,624). Avidin coated surfaces or reaction vessels prepared in this way can be used for assays where one or more of the specific assay components is labeled with biotin.

### Example 2

### Folate Assay using Polylysine-Biotin (PLB)/Avidin and Avidin Microwells

AMERLITE polystyrene microwells were coated with polylysine-biotin (PLB) and avidin as described in Example 1. Microwells, coated only with avidin, with a similar biotin binding capacity to the PLB-avidin coated wells, were compared as a control.

Sample Treatment: 10µl of serum samples, containing different concentrations of folate, and 10µl of sample treatment solution, containing 1% ascorbic acid and 0.03% dithiothreitol (DTT) were added to wells and incubated for 15 minutes at 37°C with shaking on an AMERLITE Incubator. Following this, 10µl of 0.8M NaOH (denaturant solution) was added and the wells incubated for a further 5 minutes at 37°C. Note that all treatment and denaturant solutions are aqueous solutions.

Folate Assay: After this incubation, 100µl of 0.04mg/mL biotinylated folate binding protein (biotin-FBP) and 50µl of 3ng/mL folate labeled with horseradish peroxidase (folate-HRP) in borate buffers (pH 7.2) were added and the microwells were incubated for 60 minutes at 37°C. Folate in a given sample will compete with folate-HRP for binding by biotin-FBP. The biotin-FBP/folate and biotin-FBP/folate-HRP complexes are immobilized on the microwell via the biotin avidin link. In the absence of folate in a sample, all the folate bound by biotin-FBP will be labeled with HRP, so that maximum HRP activity is immobilized on the microwell. As the concentration of folate in a given sample increases there is increasing competition with the folate-HRP for binding by biotin-FBP, such that the higher the folate concentration in the sample, the lower the HRP activity immobilized on the microwell.

After washing on an AMERLITE Washer, AMERLITE Signal Reagent was added to each well and the light output determined by an AMERLITE Analyzer. The HRP activity is measured by an enhanced luminescence reaction [Whitehead et al. (1983)]. AMERLITE Signal Reagent, containing luminogenic substrates (a luminol derivitive and a peracid salt) and an enhancer, is added to the wells to initiate the light emitting reaction. The purpose of the enhancer (a substituted phenol) is to increase the level of light produced and prolong its emission. The results of the assay are given below in Table 1.

**Table 1:**

| Folate Concentration | Mean Signal Level (arbitrary light units) | |
|---|---|---|
| (ng/mL) | PLB/Avidin Wells | Control Wells |
| 0.0 | 22.0 | 11.5 |
| 0.25 | 19.9 | 11.6 |
| 0.5 | 21.3 | 11.9 |
| 0.75 | 19.3 | 11.9 |
| 1.0 | 19.0 | 11.5 |
| 1.5 | 17.4 | 11.8 |
| 2.0 | 17.7 | 11.9 |
| 3.0 | 15.8 | 10.6 |
| 4.0 | 14.1 | 9.3 |
| 5.0 | 12.2 | 8.9 |

### Example 3

### Vitamin B12 Assay using Polylysine-Biotin (PLB)/Avidin and Avidin MicroWells

AMERLITE polystyrene microwells were coated with polylysine-biotin (PLB) and avidin as described in Example 1. Microwells, coated only with avidin, with a similar biotin binding capacity to the PLB-avidin coated wells, were compared as a control.

Sample Treatment: 10µl of serum samples, containing different concentrations of vitamin B12, and 20µl of sample treatment/denaturant solution containing 0.015 % KCN, 0.1% dithiothreitol (DTT) and 0.5M NaOH were added to wells and incubated for 15 minutes at 37°C with shaking on an AMERLITE Incubator.

Vitamin B12 Assay: After this incubation, 100µl of 5ng/mL biotinylated intrinsic factor (biotin-IF) and 50µl of 2ng/mL vitamin B12 labeled with horseradish peroxidase (vitamin B12-HRP) in phosphate buffer (pH 6.4) were added and the microwells incubated for 60 minutes at 37°C. Vitamin B12 in a given sample will compete with vitamin B12-HRP for binding by biotin-IF. The biotin-lF/vitamin B12 and biotin-lF/vitamin B12-HRP complexes are immobilized on the solid phase via the biotin avidin link. In the absence of vitamin B12 in a sample, all the vitamin B12 bound by biotin-IF will be labeled with HRP so that maximum HRP activity is immobilized on the microwell. As the concentration of vitamin B12 in a given sample increases there is increasing competition with the vitamin B12-HRP for binding by biotin-IF such that the higher the concentration in the sample, the lower the HRP activity immobilized on the microwell.

After washing on an AMERLITE Washer, AMERLITE Signal Reagent was added to each well and the light output determined by an AMERLITE Analyzer. The HRP activity is measured by an enhanced luminescence reaction as in Example 2. The results of the assay are given below in Table 2.

**Table 2:**

| Vitamin B12 Conc. (pg/ml) | Mean Signal Level (arbitrary light units) | |
|---|---|---|
| | PLB/Avidin Wells | Control Wells |
| 0 | 11.61 | 6.94 |
| 65 | 10.29 | 6.47 |
| 195 | 7.21 | 4.15 |
| 396 | 4.06 | 2.45 |
| 898 | 2.67 | 1.52 |
| 1838 | 1.19 | 0.48 |

### Results of Folate and Vitamin B12 Assays

The results, given in Tables 1 and 2, show that the use of PLB/avidin coated microwells gives significantly greater signal in the absence of folate and Vitamin B12 and a larger decrease in signal as the concentration of analyte increases compared to the avidin control wells. The differences seen are attributable to loss of biotin-binding capacity in the control wells following the sample denaturation treatment.

### Cited Literature

1. U.S. Patent No. 5,367,624.
2. Branagan, P., *Kit Inspection Laboratory Practice* 40:11, pp. 27-30 (1991).
3. Christenson, R.H., Dent, G.A and Tuszynski, A., "Two Radioassays for Serum Vitamin B12 and Folate Determination Compared in a Reference Interval Study," *Clin. Chem.* 31:8, pp. 1358-1360 (1985).
4. Bayer, E.A. and Wilchek, M. "The Avidin-Biotin Complex in Bioanalytical Applications," *Anal. Chem.* vol. 171, pp. 1-32 (1988).
5. Whitehead, T.P. et al., "Enhanced Luminescence Procedure For Sensitive Determination of Peroxidase-labeled Conjugates In Immunoassay," *Nature* vol. 305, pp. 158-9 (1983).

## Claims

1. A method for an assay for determining the presence of an analyte in a sample wherein an analyte releasing step is employed, comprising: providing a surface comprising a support coated with a molecule coupled to biotin or a biotin mimic to form a complex, wherein the complex is resistant to the analyte releasing step, and wherein said analyte releasing step and the assay are performed on the same surface.

2. The method of claim 1, wherein the molecule is a polyamine.

3. The method of claim 2, wherein the polyamine is polylysine.

4. The method of any one of claims 1 to 3, wherein the biotin or biotin mimic is further coupled to a biotin binding molecule selected from the group consisting of avidin or streptavidin.

5. The method of any one of claims 1 to 4 wherein the analyte is vitamin B12.

6. The method of any one of claims 1 to 4 wherein the analyte is folate.

## Patentansprüche

1. Verfahren für einen Test zum Bestimmen des Vorhandenseins eines Analyten in einer Probe, wobei ein Schritt verwendet wird, der einen Analyten freisetzt, umfassend: zur Verfügung stellen einer Oberfläche, die einen Träger umfaßt, beschichtet mit einem Molekül, das an Biotin oder an einen Biotin-Imitator gebunden ist, um einen Komplex zu bilden, wobei der Komplex gegenüber dem Analyten-freisetzenden Schritt widerstandsfähig ist, und wobei der Analyt-freisetzende Schritt und der Test auf der selben Oberfläche durchgeführt werden.

2. Verfahren nach Anspruch 1, wobei das Molekül ein Polyamin ist.

3. Verfahren nach Anspruch 2, wobei das Polyamin Polylysin ist.

4. Verfahren nach einem der Ansprüche 1 bis 3, wobei das Biotin oder der Biotin-Imitator weiterhin an ein Biotin-bindendes Molekül gebunden ist, ausgewählt aus der Gruppe bestehend aus Avidin oder Streptavidin.

5. Verfahren nach einem der Ansprüche 1 bis 4, wobei der Analyt Vitamin B12 ist.

6. Verfahren nach einem der Ansprüche 1 bis 4, wobei der Analyt Folat ist.

## Revendications

1. Procédé d'analyse pour déterminer la présence d'un analyte dans un échantillon dans lequel on emploie une étape de libération de l'analyte, comprenant : la fourniture d'une surface comprenant un support revêtu d'une molécule couplée à la biotine ou à un analogue de la biotine pour former un complexe, dans lequel le complexe est résistant à l'étape de libération de l'analyte, et dans lequel ladite étape de libération de l'analyte et l'analyse sont réalisées sur la même surface.

2. Procédé selon la revendication 1, dans lequel la molécule est une polyamine.

3. Procédé selon la revendication 2, dans lequel la polyamine est une polylysine.

4. Procédé selon l'une quelconque des revendications 1 à 3, dans lequel la biotine ou l'analogue de la biotine est en outre couplé à une molécule liant la biotine choisie dans le groupe constitué de l'avidine et de la streptavidine.

5. Procédé selon l'une quelconque des revendications 1 à 4, dans lequel l'analyte est la vitamine B12.

6. Procédé selon l'une quelconque des revendications 1 à 4, dans lequel l'analyte est un folate.
